# EUROPEAN PATENT APPLICATION

(11) **EP 3 070 099 A1**
(43) Date of publication of application: **21.09.2016**
(21) Application number: 16160351.9
(22) Date of filing: 15.03.2016
(51) Int. Cl.: C07K 14/535

(54) **A PROCESS FOR PREPARING GRANULOCYTE COLONY STIMULATING FACTOR (G-CSF)**

(30) Priority: 16.03.2015 TR 201503097; 13.04.2015 TR 201504463
(71) Applicant: Arven Ilac Sanayi Ve Ticaret A.S., Istanbul 34460 (TR)
(72) Inventor: YENICE, Irem, 34460 Istanbul (TR); ÜZGÜN, Mehmet, 34460 Istanbul (TR); GÜLER, Nehir, 34460 Istanbul (TR); GÜLTEPE, Idil, 34460 Istanbul (TR)
(74) Representative: Sevinç, Erkan

(57) **Abstract**

The present invention is related to a novel process of isolating and purifying granulocyte colony stimulating factor (G-CSF) from a G-CSF-producing microorganism, more specifically G-CSF is recombinant methionyl human G-CSF (rmetHuG-CSF).

## Description

### Technical Aspect

The present invention is related to a novel process of isolating and purifying granulocyte colony stimulating factor (G-CSF) from a G-CSF-producing microorganism, more specifically G-CSF is recombinant methionyl human G-CSF (rmetHuG-CSF).

### Background of the Invention

Granulocyte colony stimulating factor (G-CSF) is a 175 amino acid protein manufactured by recombinant DNA technology. G-CSF is produced by Escherichia coli (E coli) bacteria into which has been inserted the human granulocyte colony-stimulating factor gene. The protein has an amino acid sequence that is identical to the natural sequence predicted from human DNA sequence analysis, except for the addition of an N-terminal methionine necessary for expression in E coli. Because G-CSF is produced in E coli, the product is nonglycosylated and thus differs from G-CSF isolated from a human cell.

G-CSF is supplied in either vials or in prefilled syringes. The product is available in single use vials and prefilled syringes. The single use vials contain either 300 mcg or 480 mcg G-CSF at a fill volume of 1, 0 ml or 1, 6 ml respectively. The single use prefilled syringes contain either 300 mcg or 480 mcg G-CSF at a fill volume of 0, 5 ml or 0, 8 ml respectively.

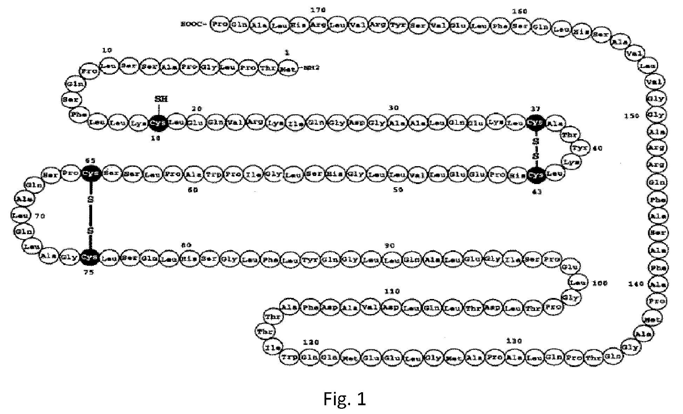

The amino acid sequence of human G-CSF is shown in Figure 1. The first amino acid of the mature protein was determined by direct amino acid sequencing of the purified proteins. Human G-CSF mRNAs code for a 30 amino acid hydrophobic leader sequence typical of secreted proteins. G-CSF has two disulphide bonds formed by homologous cysteine residues (Figure 1) and an extra cysteine residue at position at 17 that cannot participate in intramolecular disulfide bonds. The disulphide bonds in these molecules stabilize the structures and make them resistant to relatively harsh treatment (some proteases, high temperatures, denaturing solvents, extreme pH), which do lead to denaturation after reduction of disulfide bonds (Nicos A. Nicola, The walter and Eliza Hall Institute of Medical Research, "Granulocyte Colony Stimulating Factor", p. 77-100) Human G-CSF can be produced in eukaryotic organisms (yeast and mammalian cell lines) and prokaryotic organism like bacteria. The form of G-CSF is produced depends on the type of host organism used for expression. If the G-CSF is expressed in eukaryotic cells, it is produced in a soluble form and secreted. When G-CSF is produced in prokaryotic cells, the product is formed as inactive inclusion bodies (IBs). Normally inclusion bodies have a secondary structure and are densely aggregated. It is revealed that the combination of so many factors of physiological state of host cell and growth conditions is affected by the formation of inclusion bodies.

G-CSF produced in prokaryotic cells such as those of E.coli, is in inclusion bodies. Inclusion bodies are nuclear or cytoplasmic aggregates of stainable substances, usually proteins. The purification of the expressed proteins from inclusion bodies usually requires two main steps; extraction of inclusion bodies from the bacteria followed by the solubilisation of the purified inclusion bodies.

Production of biologically active human G-CSF protein from inactive inclusion bodies expressed by rDNA technology in commonly used prokaryotic host cells is very difficult. Efficient process methodologies are desirable for manufacture of therapeutically useful G-CSF on industrial scale. Various methods have been reported in scientific literature for the production of G-CSF in E.coli, yeast or mammalian CHO (Chinese hamster ovary) cells.

Many patents describe various aspects of expression and purification of G-CSF protein from different expression systems like prokaryotic and eukaryotic cells. Expression of G-CSF in bacterial systems is described in United States patents, US 4 810 643 (03.03.1986, Kirin-Amgen Inc.) and US 4 999 291 (23.08.1985, Amgen Inc.). In the preferred processes, G-CSF gene is synthesized, cloned and transformed into in E.coli. The protein is expressed by inducing the culture by elevating the temperature up to 42°C. Conditions and parameters for cloning and expression by fermentation are not clear. A number of steps are involved in clone construction. The process is performed under flask conditions and the level of protein expression is 3 - 5% only on total cellular protein basis.

The pharmaceutical product called NUPOGEN®, a trademark of Amgen Inc., includes a 175 amino acid protein manufactured by recombinant DNA technology. The product information retrievable from the web site at:
http://dailvmed.nlm.nih.aov/dailymed/archives/fdaDruaInfo.cfm?archiveid=10056 discloses that NEUPOGEN® is produced by Escherichia coli (E.coli) bacteria into which has been inserted the human granulocyte colony-stimulating factor gene. The formulation of the product contains Filgrastim, Acetate, Sorbitol, Polysorbate 80, Sodium and Water in a form suitable for intravenous and subcutaneous administration.

WO 2008/096370-A2 discloses a process for isolating and purifying G-CSF protein from a G-CSF producing E. coli comprising lysing the microorganism and separating G-CSF, isolating inclusion bodies comprising G-CSF, solubilizing G-CSF with a concentrated chaotropic agent and a reducing agent such as DTT, oxidising G-CSF, refolding - concentrating and desalting the same, subjecting it to ion exchange chromatography, and recovering purified G-CSF in a stable form.

GETZEBET AL: "A comparison between the sulfhydryl reductants tris (2-carboxyethyl) phosphine and dithiothreitol for use in protein biochemistry." Analytical Biochemistry 15 AUG 1999 LNKD-PUBMED: 10452801, vol. 273, no. 1, pages 73-80 presents a comparison between dithiothreitol (DTT) and tris(2-carboxyethyl)phosphine (TCEP) in terms of: (1) stability at neutral pH, (2) ability to preserve enzymatic activity, (3) interference with attachment of labels to protein thiols, (4) reduction of nitroxide spin probes, and (5) ability to cause unwanted protein degradation at elevated temperatures used in gel electrophoresis preparations. TCEP is however not proposed for increasing the process yield by efficiently breaking disulphide bonds with an unfolding activity in a procedure, for instance for production of granulocyte colony stimulating factor (G-CSF).

EP 0 220 520 (30.09.1985, Chugai Seiyaku Kabushiki Kaisha), WO 87/01132 (23.08.1985, Kirin-Amgen Inc.) and WO 88/01297 (11.08.1986, Cetus Corporation) describe the cloning of G-CSF.

United States patent, US 5 055 555 (05.01.1989, Sassenfeld, Helmut) describes a simplified process for production of human G-CSF from eukaryotic cells. Achieving protein secretion through extracellular eukaryotic organisms forms an entirely different technology in comparison with protein expression through intracellular prokaryotic organisms. Although the process described in patent US 5,055, 555 is simpler; it is not readily applicable to recombinant G-CSF expressed in E. coli or other cells where G-CSF is produced in the form of an inclusion body. The above method was developed for soluble G-CSF expressed and secreted by recombinant yeast into the fermentation medium. For E. coli derived G-CSF, solubilization of inclusion bodies and refolding of G-CSF are additional steps to be taken into account. Besides, obtaining a therapeutic grade preparation of GCSF free of lipopolysaccharide endotoxins, which are likely contaminants when E. coli is used as a host, in a simple, scaleable procedure has not been reported so far. On a commercial scale, yield losses from a multi-step process become highly significant. Hence a simplified procedure with fewer steps will give higher yields in a shorter time, besides being economical.

The production of recombinant therapeutic proteins using Escherichia coli as host system is viable if one can set up a simple and cost-effective downstream process. The high-level expression of eukaryotic proteins in E. coli often leads to formation of insoluble IBs in the cytoplasm. IBs formation may occur as a consequence of intracellular accumulation of partially unfolded forms of the recombinant protein. Protein properties such as the average charge, turn-forming residue fraction, cysteine and proline fractions, hydrophilicity, and total number of residues have been correlated with inclusion body formation. Culture conditions such as temperature, pH, and nutrient supply play a vital role in controlling the partition of the recombinant protein into soluble and insoluble fractions. It is often difficult to recover the protein from IBs due to the problems associated with the initial recovery, solubilization and renaturation steps.

The various purification protocols discussed in the above patents mention multiple chromatography and other steps for the purification of G-CSF. None of the above-related patents disclose a simpler, economical and commercially method for the production of G-CSF at a commercially viable scale which guarantees the stability of the product obtained by the process. The processes described in prior art are complex, lengthy and unit costs are high.

Therefore, to overcome the major problems associated with the manufacture of G-CSF, a simple, economical, industrially applicable and more stable process for high recovery of G-CSF has been now developed and disclosed in this present invention.

### Summary of the Invention

The present invention relates to a novel process of isolating and purifying granulocyte colony stimulating factor (G-CSF) from a G-CSF-producing microorganism and describes an industrially applicable, simple, cost-effective, time-saving method of producing granulocyte colony stimulating factor (G-CSF).

The main object of the present invention is to provide a novel process for isolating and purifying granulocyte colony stimulating factor (G-CSF) from a G-CSF-producing microorganism consisting the following steps;
a) harvesting the microorganisms by filtration system by adjusting particle size fraction and separating insoluble material comprising G-CSF,
b) isolating inclusion bodies by using Tangential Flow Filtration (TFF),
c) solubilizing the G-CSF present in the insoluble material, using a high concentrated chaotropic agent and a denaturing agent which is tris(2-carboxyethyl)phosphine HCl (TCEP),
d) oxidizing the G-CSF in the presence of cystine/cysteine wherein the molar ratio is 1:10,
e) refolding the G-CSF in temperature controlled conditions by a one step method,
f) optionally, concentrating the refolded G-CSF solution by using Tangential Flow Filtration (TFF) system using 5-12 KDa molecular weight cut off cassettes or cartridges,
g) addition of salt,
h) subjecting the G-CSF solution to hydrophobic interaction chromatography,

i) diafiltration,
j) subjecting the G-CSF solution to cation exchange chromatography,
k) recovering purified G-CSF in formulation buffer in the stable form,
I) optionally, removing endotoxin by using strong-basic anion exchange chromatography.

In one embodiment, the G-CSF is recombinant methionyl human G-CSF (rmetHuG-CSF).

### In one embodiment of the invention, the filtration system is microfiltration.

Another embodiment of the invention is the particle size of fraction is between 0, 1 µm and 0, 5 µm in the filtration system.

In one embodiment of the invention, the isolating the inclusion body comprising at least one washing step with buffer and at least one washing step with purified water.

Another embodiment of the invention is the buffer in the washing step comprising dithiothreitol, tris hydrochloride, ethylene diamine tetra acetic acid and sodium deoxycholate.

In one embodiment, the high concentrated chaotropic agent in the solubilizing step is guanidinium.HCl in the concentrations between 5.4 M to 6.6 M, preferably it is 6M.

In one embodiment, the temperature in the refolding step is between 7 to 15°C. According to another embodiment, the salt is selected from the group comprising NaCl, Na2HPO4, (NH4)2SO4, KCl, CH3COONH4, K2SO4 and Na2SO4.

In one embodiment, the salt is NaCl in concentration between 1 M to 3M.

In one embodiment, the hydrophobic interaction chromatography comprising column having hydrophobic ligands.

Another embodiment of the invention is hydrophobic ligand is selected from the group comprising phenyl, butyl or oclyl functional groups.

In one embodiment, the cation exchange chromatography comprises sulfopropyl.

In another embodiment of invention the hydrophobic interaction chromatography elution buffer is an aqueous solution of 20 mM Acetate buffer, %5 Sorbitol and 2 M Urea adjusted to pH 5.4.

Another embodiment the stable formulation buffer in the recovering step is an aqueous solution of 10 mM Acetate buffer, %5 Sorbitol and %0.004 Polysorbate 80 adjusted to pH 4.0.

In another embodiment, the strong-basic anion exchange chromatography step used in removing the endotoxins is consisting of quaternary ammonium.

In one embodiment, in step c) the pH is from 7.5 to 9.0, preferably it is 8.

In another embodiment, in step e) the pH is from 7.0 to 8.0, preferably it is 7.5.

According to another embodiment, G-CSF producing microorganisms is E.coli.

In a further embodiment of the invention, the method comprises the step of formulating the rmetHuG-CSF for producing a biopharmaceutical product consisting of;
a) 30 - 100 MU (300 - 1000 µg) of rmetHuG-CSF
b) 0,5 - 0,80 mg of acetate (or 0,250 - 1,00 mg sodium acetate)
c) 25 - 100 mg of sorbitol
d) 0,01 - 0,10 mg of polysorbate 80
e) water for injection
f) sodium hydroxide or acetic acid for pH adjustment
in 1 ml sterile solution which is acceptable for filling into appropriate delivering devices of defined dosage strengths for administration to patients.

In a preferred embodiment of acetate is 0.56 mg.

Biopharmaceutical products obtained by using the process of the present invention can be useful in the treatment of neutropenia, leukaemia, mobilization of peripheral blood progenitor cells (PBPC), HIV infections such as bacterial infections and cancer patients receiving cytotoxic chemotherapy, myelosuppressive chemotherapy and bone marrow transplant.

In prior art, different type of the microorganisms, different type of strain and also different modified gene sequences to produce of the G-CSF are described. Each process has specific production conditions and their problems that can be obtained from the process conditions or produced target protein or impurities. Development of the purification steps in protein production depends on the un-purified protein specifications obtained from up-stream process in different conditions. The present invention is related to production of the pure and stable G-CSF (98% according to "size exclusion method" of EP) as a final product considering of the time, labor and cost consuming. The processes available to date are generally time, labor and cost consuming as well as being complicated. Moreover, they fail to provide stable results. There is still a need for improved purifications results.

### Detailed Description of the invention

A simple and innovative method for the production of G-CSF which is recombinant methionyl human G-CSF (rmetHuG-CSF) is carried out by using E.coli cells due to the high level of protein expression and avoidance of glycosylation complications.

E.coli cells are harvested after fermentation by filtration system and the proteinaceous material, which is in the form of inclusion body, is extracted from cells by lysing the bacterial cells using a high pressure jet sprayer by adjusting particle size fraction and separating insoluble material comprising G-CSF. Bacterial cell wall is broken by physical means thus enabling inclusion bodies in the cells to suspend freely in buffer. Inclusion body (IB) from cell disruption process is washed with diafiltration method. After washing and filtration steps, inclusion bodies are solubilized in a suitable solubilization buffer containing a high concentrated chaotropic agent. The solubilization buffer is aqueous solution with pH value of 8.0 comprising 6M Guanidine.HCl as chaotropic agent and 100mM TRIS as buffering salt. Inclusion bodies are solubilized with the buffer mentioned above at room temperature.

### Purification

After solubilization step of inclusion bodies, there are several different proteinaceous materials, not only different from G-CSF but also several isoforms of G-CSF. There is a need to change the conformation of misfolded G-CSF and break intermolecular disulphide bridges which may form dimers, oligomers and aggregates. Thus, solubilized proteins with a proper denaturing agent are denatured to obtain unfolded monomer chains. The denaturation step of the proteins is important and it affects the final product yield and purity. If misfolded protein remains in the solution it may trigger aggregation trough all purification steps. Thus 0.5M tris-2-carboxyethylphoshpine.HCl (TCEP) or 0.5M DTT is chosen as a strong denaturing agent to obtain desired denaturation levels. In 1-2 hours of mixing at room temperature enables proper levels of denaturation.

DTT is an odorous chemical and when it contacts with the air, it can be easily oxidised. Due to air oxidation it is difficult to store DDT as a solvent and it is relatively unstable compound. In this invention TCEP is used as a strong denaturing agent.

Refolding of G-CSF molecules is conducted in the presence of a pair of oxidizing/reducing agent. Cystine/cysteine couple is used to obtain properly folded G-CSF molecule formation. The molar ratio of cystine to cysteine is 1/10. Refolding solution is aqueous solution with pH value 7.5 comprising 0.5 M L-Arginine.HCl, 50 mg/ml Sorbitol, 0.1M TRIS and 2mM EDTA. Refolding solution volume is adjusted to reach 0.2mg/ml concentration of denatured protein. The solution is kept in between 7 to 11°C more preferably 8 to 10°C for 16-20 hours to reach proper folding conformation and desired folding levels.

Tangential Flow Filtration (TFF) is used to remove undesired molecules which have molecular weight smaller than 5-12kDa. By this way, not only solution is concentrated but also some process related impurities such as small protein molecules which could not removed in previous steps could be removed. In this present invention, optionally concentrating the refolded G-CSF solution by using Tangential Flow Filtration (TFF) system using 5-12 KDa molecular weights cut off cassettes or cartridges.

The addition of salt makes the protein more hydrophobic and enables to use Hydrophobic Interaction Chromatography to bind the protein to a hydrophobic interaction media. Thus, salt addition prepares the protein solution for the next step which is hydrophobic interaction chromatography. Most commonly used salts are Na2HPO4, (NH4)2SO4, KCl, CH3COONH4, K2SO4, Na2SO4 and NaCl in concentration between 1 M to 3 M. Preferably, NaCl is used in concentration between 1 M to 2M. Sodium chloride is added to the refolding solution at 12ºC, and mixed at a low rate in order to dissolve. The concentration of sodium chloride should be 2M at the end of dissolving.

The method of hydrophobic interaction chromatography (HIC) is based on the observation that protein molecules can interact with fully hydrophobic adsorbents, and this interaction is dependent on the salt concentration of the solution. Increasing salt concentration will lead to the aggregation and precipitation of protein molecules via the rearrangement of their hydrate shell, during HIC chromatography a high salt concentration facilitates the interaction between the hydrophobic chromatographic medium and the hydrophobic patches present on protein molecules. During separation, the decrease in salt concentration will lead to the elution of bound molecules.

Based on these observations, bead polymers were created that are suitable for HIC chromatographic purposes. Suitable HIC matrices may include, but are not limited to, alkyl- or aryl-substituted matrices, such as butyl-, hexyl-, octyl- or phenyl-substituted matrices including agarose, cross-linked agarose, sepharose, cellulose, silica, dextran, polystyrene, poly (methacrylate) matrices, and mixed mode resins. Such media include the polysaccharide-based Butyl-Sepharose, Octyl-Sepharose and Phenyl-Sepharose materials that are derived from polymers that had proven to be suitable for chromatographic separation of proteins. Preferably they are Phenyl-Sepharose.

Hydrophobic interaction chromatography is unique in that proteins bind at high salt concentration and elute at low salt concentration. Adsorption of the protein of interest to a HIC column is favored by high salt concentrations, but the actual concentrations can vary over a wide range depending on the nature of the protein of interest, salt type and the particular HIC ligand chosen. Examples of salts are ammonium acetate, ammonium sulphate, Disodium phosphate sodium sulfate, sodium chloride, potassium chloride and potassium sulphate. Preferred salt is sodium chloride in the concentrations of 1.5 to 3M, preferably it is 2M.

Many proteins including G-CSF can form aggregates that must be removed during purification in order to provide a protein product having the required purity and for therapeutic proteins product safety. Although aggregate removal is a key determinant of product safety, it may increase the difficulty of process development and increase down-stream process cost.

There are various patent application in prior art in relation to process for preparing G-CSF, for example, EP 2 341 061 describes the process with desalting chromatography step. The G-CSF is separated from said salt by the huge molecular size difference by injection into and moving in the desalting column. In scale-up applications of this step, the protein is highly susceptible to aggregation. Moreover aggregation problem causes permanent column problems and low yield. Therefore elimination of aggregates from bulk product in an efficient manner is need for successful production of high strength biopharmaceutical.

In this novel invention, it is surprisingly found that the process comprising HIC step provides advantages to prevent aggregation. Preventing aggregation provides to shorten the time of the process and increase the yield. It greatly extends the life of column and helps to obtain its best performance. In addition, it provides to ease the process validation. In large scale production using of HIC step in the process is efficient and it provides robust purification methods for G-CSF

In certain embodiments, the purification strategies of the present invention may include one or more additional chromatography and/or filtration steps after the HIC purification step.

The concentration of the salt in the eluate obtained from HIC step should be decreased prior to Cation Exchange Chromatography, since the ionic strength of salt will interfere with protein's binding to the Cation Exchange resin. Therefore, protein solution salts collected from hydrophobic interaction chromatography column are removed at this stage. Appropriate TFF cassettes and suitable mobile phase is used for this step. Thus G-CSF is taken into less salty buffer. Removal of the salt is monitored by conductivity measurements. The process takes place at room temperature.

The diafiltration step is conducted using a 5 - 12 kDa casettes. Preferably 10 kDa casettes are used at this stage. Mobile phase is used to remove salts with diafiltration. Diafiltration is continued until conductivity of the solution is below 1.8 mS/cm.

The outlet solution of diafiltration is then subjected to cation exchange chromatography. This is the step in which properly folded G-CSF is removed from its misfolded isoforms and other impurities. The proteins are first fed to a cation exchange column filled with strong cation exchange material and they are hold by the media. Then gradually using a gradient method, the conductivity is increased to certain values enabling the impurities and G-CSF to elute on different times for separation with respect to their different pl values. Gradient method utilizes usage of two different mobile phases of two different conductivity values: mobile phase A and mobile phase B. Mobile phase A is a pH=5.4 solution, having conductivity value 0.5 - 1.5mS/cm, comprising 20mM Sodium Asetate and 50mg/ml Sorbitol. Mobile phase B is a pH=5.4 solution, having conductivity value 15-25 mS/cm, comprising 20mM Sodium Asetate, 50mg/ml Sorbitol and 0.2M NaCl.

Pure protein material present in the buffered water solution is the subjected to gel filtration to produce the final product. Thus, active G-CSF molecule is directly recovered as final product with a proper and stable formulation avoiding complexity and problems of other recovery techniques, such as lyophilization and reconstitution. Gel filtration is carried out with the same matrix in desalting step but with different mobile phase composition, which is named as formulation buffer. It comprises 10mum Sodium Acetate and 50mg/ml Sorbitol and %0.004 Polysorbate 80 with pH value 4.0

Endotoxin level is an optional part of quality control testing in producing sterile injectable solution such as the product which is produced by present invention. In production biological and biotechnological active substances, sterile solutions such as washing buffers, mobile phases and formulation solutions can be very costly which may also require a controlled environment that will also contribute to high cost by its requirement for regular microbiological testing and control. It is a challenge to keep endotoxin levels if a fully controlled sterile environment is not available.

The present invention allows the production costs drop significantly by making it possible to work in a non-sterile environment and keep the endotoxin levels comply with EP monograph. The present invention utilizes ion exchange chromatography in which ion exchange media is a strong basic anion exchanger. The strong anion exchange media can be quaternary ammonium. By utilization of ion exchange chromatography with strong basic anion exchangers, the endotoxin levels can be dropped much below.

Large scale purification of proteins remains a significant challenge in the biopharmaceutical industry, as efficient and cost-effective methods are required to achieve desired yields and purity levels.

The present invention provides an improved method for aseptic purification of G-CSF in lab-, pilot-, and, especially in industrial-scale. The invention provides a robust downstream purification process suitable for use in manufacturing of G-CSF for human administration. One of the important challenges is the development of efficient and competent process for the large scale purification of G-CSF.

In a preferred embodiment of the invention, biologically active G-CSF obtained by using the process of the present invention can be used for preparation of medicaments.

According to this embodiment of the invention, one of the medicament is the biopharmaceutical product obtainable by the process of the described invention is consisting of
a) 30 - 100 MU (300 - 1000 µg) of rmetHuG-CSF
b) 0,50 - 0,80 mg of acetate (or 0,250 - 1,00 mg sodium acetate)
c) 25 - 100 mg of sorbitol
d) 0,01 - 0,10 mg of polysorbate 80
e) water for injection
f) sodium hydroxide or acetic acid for pH adjustment
in 1 ml sterile solution.

Such medicaments are indicated for a wide range of indications and include; neutropenia and neutropenia-related clinical sequelae, chronic neutropenia, neutropenic and non-neutropenic infections, reduction of hospitalisation for febrile neutropenia after cytotoxic chemotherapy and for the reduction in the duration of neutropenia in patients undergoing myeloablative therapy followed by bone marrow transplantation considered to be at increased risk of prolonged severe neutropenia.

G-CSF is also indicated for mobilisation of peripheral blood progenitor cells (PBPC) and chronic inflammatory conditions.

Its long term administration is indicated to increase neutrophil counts and to reduce the incidence and duration of infection-related events, treatment of persistent neutropenia in patients with advanced HIV infection, in order to reduce the risk of bacterial infections.

G-CSF is also indicated for improving the clinical outcome in intensive care unit patients and critically ill patients, wound/skin ulcers/burns healing and treatment, intensification of chemotherapy and/or radiotherapy, increase of anti-inflammatory citokines, potentiation of the antitumour effects of photodynamic therapy.

It is indicated for prevention and treatment of illness caused by different cerebral disfunctions, treatment of thrombotic illness and their complications and post irradiation recovery of erythropoiesis. It can be also used for treatment of all other illnesses, which are indicative for G-CSF.

A biopharmaceutical composition containing the pure and biologically active G-CSF obtained by the process of the invention can thus be administered, to patients, children or adults in a therapeutically amount which is effective to treat the above mentioned diseases.

The present invention is further described by referring to the following example. Although the example is not intended to limit the scope of the present invention, it should be considered in the light of the description detailed above.

### Example

### Hydrophobic Interaction Chromatograph

G-CSF passes through the hydrophobic interaction chromatography (HIC) column and binds to the media. The impurities that are not bound on the column will be removed. Afterwards, isocratic elution method is used in order to collect the proteins bound inside.

HIC column is packed with Phenyl Sepharose hydrophobic interaction media. The column has 25 cm diameter packed with 13.2 cm media. Its packed volume is ∼6.5 L. First stage is to bind the protein inside the column. First the column is equilibrated with 3 M Sodium Chloride solution. After the sample is injected into column, the absorbance value of the outlet solution is carefully monitored at 280 nm wave-length for absorbance. Then the protein is eluted by injecting the HIC elution mobile phase which is an aqueous solution of pH 5.4 comprising 20mM Sodium Asetate, 50 mg/ml sorbitol and 120 mg/ml Urea. The baseline each parameter is maintained until molecules are eluted. The process takes place at room temperature.

### Cation Exchange

Properly folded G-CSF is removed from its impurities and purified. Intermediate product is taken after diafiltration and fed to cation exchange column with mobile phase A which is pH=5.4, having conductivity value 1.0 - 2.0 uS/cm, comprising 20mM Sodium Asetate and 50mg/ml Sorbitol. While sample is being fed to column, 280nm UV baseline is maintained. The increase in baseline is the signal of column holding capacity. Proper column volume should be chosen to avoid column saturation. After sample is fed, purification process continues with gradual mobile phase B injection into column with a gradient program to allow protein separation and impurities. Mobile phase B is pH=5.4, having conductivity value 40 - 50 uS/cm, comprising 20mM Sodium Asetate, 50mg/ml Sorbitol and 0.2M NaCl. Its higher conductivity value enables change of salt concentration in the column to separate proteins.

### Gel Filtration for Final Step

G-CSF is recovered directly into the formulation buffer. At this step the intermediate product is in the cation exchange outlet buffer which contains 0.02-0.2 M NaCl. Since the product is IV injection the salt and the product should be separated thus gel filtration chromatography is conducted. As mentioned above, the mobile phase for this step is the formulation buffer which is aqueous solution with pH value 4.0, comprising 10mM Sodium Acetate and 50mg/ml Sorbitol and %0.004 Polysorbate 80. As the sample is injected into column, the absorbance and conductivity value of the outlet solution is carefully monitored at 280 nm wavelength for absorbance and about 1.35 umS/cm for conductivity. The baseline for each parameter is maintained until molecules are eluted. Since protein molecules are eluted first, the first observed peak is on absorbance baseline which corresponds to that the protein coming out of the column. Product is separated until an increase in conductivity is monitored which corresponds to elution of salts are taking place.

### Anion Exchange

This is the optional step in which G-CSF containing product could be further removed of its endotoxin impurities. At this step final product which is G-CSF recovered to formulation buffer as in gel filtration step, is fed to ion exchanger column continuously in a recycling manner for a number of times and at the end endotoxin free sample is sterile filtered for filling.

## Claims

1. A process for isolating and purifying granulocyte colony stimulating factor (G-CSF) from a G-CSF-producing microorganism consisting of the following steps;
a) harvesting the microorganisms by filtration system by adjusting particle size fraction and separating insoluble material comprising G-CSF,
b) isolating inclusion bodies by using Tangential Flow Filtration (TFF),
c) solubilizing the G-CSF present in the insoluble material, using a high concentrated chaotropic agent and a denaturing agent which is tris(2-carboxyethyl)phosphine HCl (TCEP),
d) oxidizing the G-CSF in the presence of cystine/cysteine wherein the molar ratio is 1:10,
e) refolding the G-CSF in temperature controlled conditions by a one step method,
f) optionally, concentrating the refolded G-CSF solution by using Tangential Flow Filtration (TFF) system using 5-12 KDa molecular weight cut off cassettes or cartridges,
g) addition of salt,
h) subjecting the G-CSF solution to hydrophobic interaction chromatography,
i) diafiltration,
j) subjecting the G-CSF solution to cation exchange chromatography,
k) recovering purified G-CSF in formulation buffer in the stable form,
l) optionally, removing endotoxin by using strong-basic anion exchange chromatography.

2. The process according to claim 1, wherein the G-CSF is recombinant methionyl human G-CSF (rmetHuG-CSF).

3. The process according to claim 1 and 2 wherein the filtration system is microfiltration.

4. The process according to claim 1 and 2 wherein the particle size of fraction is between 0, 1 µm and 0, 5 µm in the filtration system.

5. The process according to claim 1 and 2 wherein the isolating comprising at least one washing step with buffer and at least one washing step with purified water.

6. The process according to claim 5 wherein the buffer in the washing step comprising dithiothreitol, tris hydrochloride, ethylene diamine tetra acetic acid and sodium deoxycholate.

7. The process according to claims 1 and 2, wherein the high concentrated chaotropic agent in the solubilizing step is guanidinium.HCl in the concentrations between 5.4 M to 6.6 M.

8. The process according to claims 1 and 2, wherein the temperature in the refolding step is between 7 to 15 °C.

9. The process according to claims 1 and 2 wherein the salt is selected from the group comprising NaCl, Na2HPO4, (NH4)2SO4, KCl, CH3COONH4, K2SO4 and Na2SO4

10. The process according to claim 9 wherein the salt is NaCl in concentration between 1 M to 3M

11. The process according to claim 1 and 2 wherein the hydrophobic interaction chromatography comprising column having hydrophobic ligands.

12. The process according to claim 11 wherein hydrophobic ligand is selected from the group comprising phenyl, butyl or oclyl functional groups

13. The process according to claims 1 and 2, wherein the cation exchange chromatography comprises sulfopropyl.

14. The process according to claim 1, wherein in step h) the hydrophobic interaction chromatography elution buffer is an aqueous solution of 20 mM Acetate buffer, %5 Sorbitol and 2 M Urea adjusted to pH 5.4.

15. The process according to claims 1 and 2, wherein the stable formulation buffer in the recovering step is an aqueous solution of 10 mM Acetate buffer, %5 Sorbitol and %0.004 Polysorbate 80 adjusted to pH 4.0.

16. The process according to claims 1 and 2, wherein the strong-basic anion exchange chromatography step used in removing the endotoxins is consisting of quaternary ammonium.

17. The process according to claims 1 and 2, wherein in step c) the pH is from 7.5 to 9.0.

18. The process according to claims 1 and 2 wherein in step e) the pH is from 7.0 to 8.0.

19. The process according to any of the preceding claims, wherein a G-CSF-producing microorganism is E.coli.

20. The process according to claim 2, further comprising the step of formulating the rmetHuG-CSF according to a composition consisting of:
a) 30-100 MU (300 - 1000 µg) of rmetHuG-CSF
b) 0.50 - 0.80 mg of acetate
c) 25-100 mg of sorbitol
d) 0,01 - 0,10 mg of polysorbate 80
e) water for injection
f) sodium hydroxide or acetic acid for pH adjustment
in 1 ml sterile solution.
